(19) Europäisches Patentamt
European Patent Office
Office européen des brevets

(11) Veröffentlichungsnummer: 0 000 770 A2

# (12) EUROPÄISCHE PATENTANMELDUNG

(21) Anmeldenummer: 78100579.8

(22) Anmeldetag: 02.08.78

(51) Int. Cl.²: C 07 H 19/16, A 61 K 31/70
C 07 H 19/04, A 61 K 35/56

(30) Priorität: 03.08.77 LU 77910
10.02.78 US 876769
10.02.78 US 876768
13.06.78 GB 26761/78

(43) Veröffentlichungstag der Anmeldung: 21.02.79 Patentblatt 79/4

(84) Benannte Vertragsstaaten: BE CH DE FR GB LU NL SE

(71) Anmelder: F.Hoffmann-La Roche & Co. Aktiengesellschaft
Abt. VIII-Pt
CH-4002 Basel(CH)

(72) Erfinder: Gregson, Richard Peter
22 Towradgi Street
Narraweena, N.S.W.(AU)

(72) Erfinder: Quinn, Ronald James
9 Boylson Place
Cromer, N.S.W.(AU)

(72) Erfinder: Cook, Alan Frederick
19 Hillcrest Road
Cedar Grove, N.J.(US)

(74) Vertreter: Lederer, Franz, Dr. et al,
Patentanwälte Lederer, Meyer Lucile-Grahn-Strasse 22
D-8000 München 80(DE)

(54) **Neues Isoguanosinderivat, Verfahren und Zwischenprodukte zu dessen Herstellung und pharmazeutische Präparate, sowie deren Herstellung.**

(57) Die Erfindung betrifft eine bisher unbekannte Verbindung, 1-Methyl-isoguanosin, und ein Verfahren zu deren Herstellung durch Isolierung aus natürlichen Vorkommen in an sich bekannter Weise oder durch ein chemisches Verfahren. 1-Methyl-isoguanosin kann als Muskelrelaxant und/oder als Mittel zur Beeinflussung des zentralen Nervensystem und/oder als antiinflammatorisches und/oder antiallergisches und/oder hypotensives Mittel Verwendung finden.

EP 0 000 770 A2

0000770

2. Aug. 1978

RAN 4008/295-K

F. Hoffmann-La Roche & Co. Aktiengesellschaft, Basel/Schweiz

Neues Isoguanosinderivat, Verfahren und Zwischenprodukte zu dessen Herstellung und pharmazeutische Präparate, sowie deren Herstellung

Die vorliegende Erfindung betrifft eine bisher unbekannte cyclische Verbindung, nämlich das 1-Methyl-9βD-ribofuranosyl-isoguanin (1-Methyl-isoguanosin), welches in tautomeren Formen vorliegen kann, z.B. der Formel

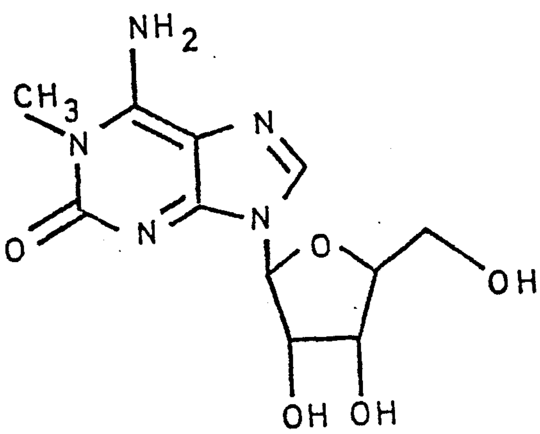

Ia

Mé/16.6.78

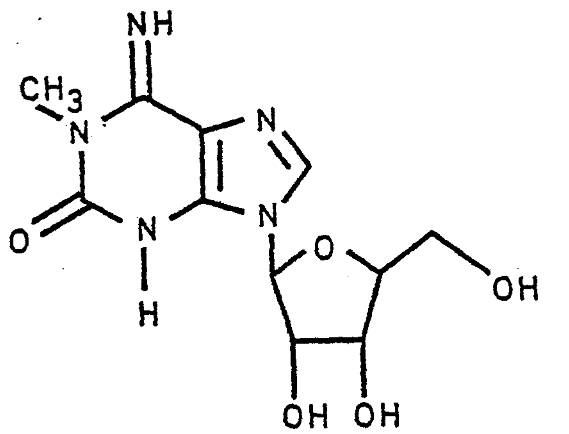

Ib

und

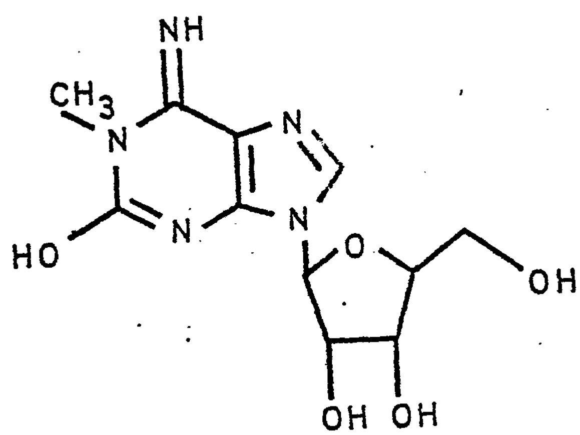

Ic

Erfindungsgemäss wurde gefunden, dass das 1-Methyl-isoguanosin in geringen Mengen in Meeresorganismen, insbesondere in Schwämmen der Gattung Tedania, welche sich an der australischen Küste finden, enthalten ist.

In einer Hinsicht betrifft die Erfindung die Isolierung von 1-Methyl-isoguanosin aus natürlichen Vorkommen, insbesondere Meeresorganismen, vor allem Schwämmen der Gattung Tedania.

Weiterhin betrifft die Erfindung das 1-Methyl-isoguanosin selbst, sowie in praktisch reiner Form, d.h. frei von natürlich vorkommenden Begleitstoffen.

1-Methyl-isoguanosin kann aus natürlichen Vorkommen, z.B. Schwämmen, in an sich bekannter Weise isoliert werden, z.B. durch Extraktion mit Dimethylsulfoxyd, Wasser und/oder einem organischen hydroxylischen Lösungsmittel, wie einem niederen Alkanol, z.B. Methanol oder Aethanol, wobei ein Aethanol-Wassergemisch bevorzugt ist, und anschliessende Chromatographie, vorzugsweise Ionenaustauscherchromatographie, z.B. mittels eines Kationenaustauscherharzes, wie einem $SO_3H$-Gruppen enthaltenden Harz und einem milden sauren Puffer, wie Ammoniumformiat.

Die Erfindung betrifft ferner ein Verfahren zur Herstellung von 1-Methyl-isoguanosin durch Umsetzung eines Imidazolderivates der Formel

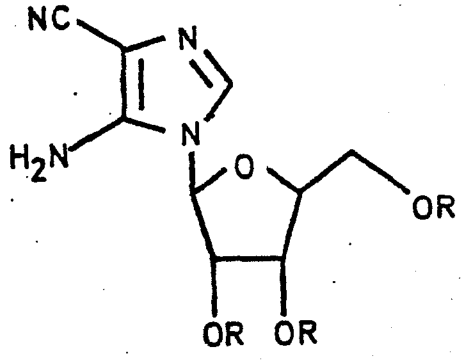

II

worin R eine leicht abspaltbare Acylgruppe darstellt,

mit Methylisocyanat in Gegenwart eines stark aprotischen Lösungsmittels, wie Dimethylformamid, Dimethylsulfoxid oder Hexamethylphosphoramid, zweckmässiger Weise bei einer Temperatur zwischen etwa Raumtemperatur und 150°C, vorzugsweise bei etwa 100°C.

Das bei dieser Reaktion entstandene neue Zwischenprodukt der Formel

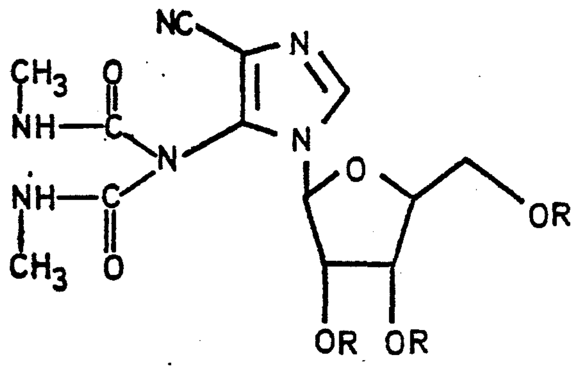

III

worin R obige Bedeutung hat, wird dann mit einer Base, vorzugsweise einer schwachen Base, wie Ammoniumhydroxid, in Gegenwart oder in Abwesenheit eines $C_{1-4}$-Alkanols, mit wasserfreiem Ammoniak in Gegenwart eines $C_{1-4}$-Alkanols, oder mit einem Alkalimetallhydroxid, zum Beispiel Natrium- oder Kaliumhydroxid umgesetzt. Zweckmässiger Weise wird diese Reaktion bei einer Temperatur zwischen etwa 0 oder 100°C, vorzugsweise bei Raumtemperatur durchgeführt.

Beispiele von leicht abspaltbaren Acylgruppen R in den Ausgangsmaterialien der Formel II sind Niederalkanoyl, wie Acetyl, Propionyl oder Butyryl, Aroyl oder substituiertes Aroyl, wie Benzoyl, p-Tolyoyl, p-Methoxybenzoyl oder p-Chlorbenzoyl, vorzugsweise Acetyl.

Ferner betrifft die Erfindung ein Verfahren zur Herstellung von 1-Methyl-isoguanosin durch Reaktion einer Verbindung der Formel

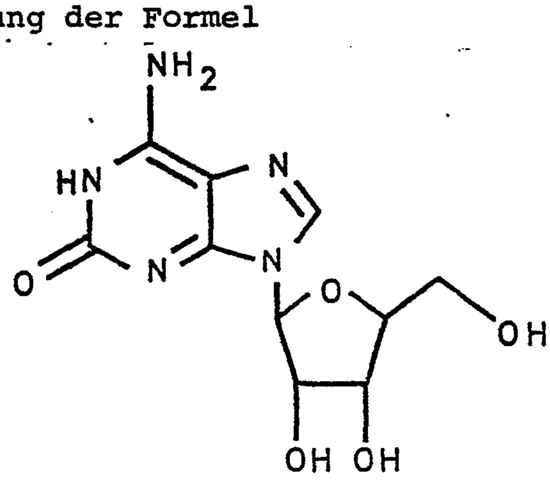

IV

mit einem Methylierungsmittel.

Die Methylierung wird vorzugsweise unter Verwendung eines Methylhalogenids, zum Beispiel Methyliodid oder Bromid, in Gegenwart eines aprotischen Lösungsmittels, wie Dimethylformamid, Dimethylsulfoxid oder Hexamethylphosphoramid, unter basischen Bedingungen, zum Beispiel in Gegenwart von Natrium- oder Kaliumhydroxid oder Carbonat, bei einer Temperatur zwischen etwa 0 und 100°C, vorzugsweise bei etwa Raumtemperatur durchgeführt. Als Nebenprodukt der obigen Methylierung wird das aus J.Org. Chem. 22, 1957, 1575 bekannte 9β-D-ribofuranosyl-2-methoxy-adenin (2-Methoxy-adenosin) erhalten. Die Produkte können in an sich bekannter Weise, zum Beispiel durch Chromatographie, getrennt werden.

Die Methylierung in 1-Stellung des Isoguanosins IV kann auch unter Verwendung von Diazomethan in einem Lösungsmittel, wie einem Aether, zum Beispiel Dioxan oder 1,2-Dimethoxyäthan, oder einem $C_{1-4}$-Alkanol, zum Beispiel Aethanol durchgeführt werden. Als Methylierungsmittel kann auch Dimethylsulfat in einem polaren aprotischen Lösungsmittel, wie Dioxan, oder in Wasser verwendet werden. Diese Reaktionen werden vorzugsweise bei etwa Raumtemperatur durchgeführt.

Erfindungsgemäss wurde weiter gefunden, dass 1-Methyl-isoguanosin verschiedene therapeutische Aktivitäten entfaltet, z.B. muskelrelaxierend wirkt, das zentrale Nervensystem beeinflusst, anti-inflammatorisch, anti-allergisch und hypotensiv wirksam ist. Bei der Maus beträgt der $ED_{50}$-Wert für 1-Methyl-isoguanosin als Muskelrelaxans bei intraperitonealer Applikation 3,1 mg/kg und bei oraler Applikation 12 mg/kg. Die $LD_{50}$ bei der Maus beträgt 1000 mg/kg p.o. nach 48 Stunden.

1-Methyl-isoguanosin kann als Muskelrelaxans und/oder als Mittel zur Beeinflussung des zentralen Nervensystems und/oder als anti-inflammatorisches und/oder anti-allergisches und/oder hypotensives Mittel Verwendung finden. Es kann in Form pharmazeutischer Präparate zusammen mit einem verträglichen pharmazeutischen Träger, z.B. einem organischen oder anorganischen inerten Träger, der für enterale, vorzugsweise orale, oder parenterale Administration geeignet ist, verwendet werden. Beispiele solcher Träger sind Wasser, Gelatine, Lactose, Stärke, Talkum, Magnesiumstearat, Gummi, vegetabile Oele und Vaseline. Die pharmazeutischen Präparate können in fester Form, z.B. als Tabletten, Kapseln, Dragées oder Suppositorien oder in flüssiger Form, z.B. als Lösungen, Emulsionen oder Suspensionen vorliegen. Sie können sterilisiert sein und/oder verträgliche Hilfsmittel, wie Konservierungsmittel, Stabilisierungsmittel, Geschmacksstoffe, Farbstoffe, Emulgatoren und Salze zur Veränderung des osmotischen Druckes oder Puffer enthalten.

Eine geeignete pharmazeutische Dosierungsform enthält etwa 1-100 mg 1-Methyl-isoguanosin. Die Dosierung für die orale Verabreichung liegt im Bereich von 0,1 mg pro kg bis 25 mg pro kg pro Tag. Für die parenterale Verabreichung kommen Dosierungen im Bereich von 0,01 mg pro kg bis 10 mg pro kg pro Tag in Betracht. Diese Bereiche können jedoch nach oben oder unten in Abhängigkeit von den individuellen Erfordernissen variiert werden.

Die folgenden Beispiele erläutern die Erfindung.

## Beispiel 1

Das Ausgangsmaterial war ein orangefarbener Schwamm der Gattung Tedania, der an der australischen Küste gesammelt wurde. Der gefrorene Organismus wurde lyophilisiert und gemahlen. Das erhaltene Pulver (300 g) wurde mit einem Aethanol: Wassergemisch (3:7 Volumteile, 2 l) 24 Stunden bei 4°C gerührt, danach filtriert und der Rückstand erneut extrahiert. Die vereinigten Extrakte wurden bei 35°C und 7 Torr auf ein Volumen von 1,2 l konzentriert, die so erhaltene wässrige Suspension 0,3 Stunden mit 13000 g zentrifugiert und der Ueberstand lyophilisiert. Man erhielt so 75,7 g eines orangefarbenen Pulvers A.

205 g Rohextrakt A wurden in 0,1 M Ammoniumformiatpuffer (pH 3,5) suspendiert, 15 Minuten beschallt und 40 Minuten auf dem siedenden Wasserbad gerührt. Die Suspension wurde auf 22°C gekühlt, das pH durch Zusatz von Ameisensäure von 4,2 auf 3,5 gebracht und sodann mit 13000 g eine halbe Stunde zentrifugiert. Der Ueberstand wurde auf eine Säule (40 x 5 cm) von Bio Rad AG50W-X8 (Ammoniumform, 200-400 Mesh) gegeben, und mit 0,1 M Ammoniumformiat (pH 3,5) mit einer Durchflussgeschwindigkeit von 150 ml pro Stunde äquilibriert. Dabei wurde die prozentuale Durchlässigkeit des Eluats bei 254 und 280 nm überwacht. Nachdem 10 l Puffer durchgesetzt waren, wurde 0,1 M Ammoniumformiat (pH 5,3) auf die Säule gegeben. Es wurden folgende Fraktionen gesammelt: 1,2 l (pH 3,5-4,8) 4,5 l (pH 4,8-5,0) und 5 l (pH 5,0-5,2). Nur die Fraktion vom pH 4,8-5,0 war aktiv, sie wurde lyophilisiert und lieferte einen hellgelben Feststoff B (31,6 g).

Eine Lösung von 68,9 g B in 1,25 l 0,1 M Ammoniumformiat (pH 3,5) wurden 20 Minuten mit 13000 g zentrifugiert. Der Ueberstand wurde mit 0,1 M Ammoniumformiat (pH 3,5) auf 4 l verdünnt und das pH wurde durch Zusatz von Ameisensäure auf 3,7 gestellt. Die Lösung wurde auf eine Säule (41,5 x 5 cm) von Bio Rad AG50W-X8 (Ammoniumform, 200-400 Mesh)

gegeben und die oben angegebene Prozedur für die Behandlung des Rohextrakts A wurde wiederholt. Nachdem 5,1 l pH 3,5 Puffer eluiert worden waren, wurde Puffer vom pH 5,3 auf die Säule gegeben und das aktive Material zwischen 7,85 l und 10,85 l eluiert. Die Lyophilisierung der aktiven Fraktion lieferte 10,8 g C.

Umkristallisation von C aus siedendem Wasser lieferte 2,75 g 1-Methyl-isoguanosin als farblosen kristallinen Feststoff, Schmelzpunkt 262-263°C, $[\alpha]^{24}_{589}$ - 65,4° (c = 1,0, Dimethylsulfoxid) $[\alpha]^{22}_{589}$ - 54,6° (c = 1,0, Wasser).

## Beispiel 2
## 1-Methyl-isoguanosin

Eine Lösung von 5-Amino-4-cyan-1-(2', 3', 5'-tri-O-acetyl-β-D-ribofuranosyl)imidazol (25,4 g, 69,3 mMol) und Methylisocyanat (40,9 ml, 693 mMol) in DMF (250 ml, getrocknet über 4A Molekularsiebe) wird unter Rühren bei 100°C erhitzt. Nach 6 Stunden wird das Gemisch abgekühlt und zu einem Oel eingedampft. Spuren von DMF werden durch Eindampfen zusammen mit 250 ml Methanol entfernt. Der entstandene Schaum wird in 250 ml Methanol gelöst, filtriert und zur Trockene eingedampft. Der Schaum wird über Nacht unter Vakuum gelassen, dann in 150 ml Methanol gelöst und über Nacht bei 5°C mit 150 ml konzentriertem Ammoniumhydroxid behandelt. Die entstandenen Kristalle werden gesammelt, mit Methanol gewaschen und aus Aethanol/Wasser (1/1) umkristallisiert. Durch wiederholte Umkristallisationen werden Spuren von Verunreinigungen entfernt. Schmelzpunkt 265-266°C, Zersetzung.

## Beispiel 3

Eine Lösung von 5 g 5-Amino-4-cyan-1-(2', 3', 5'-tri-O-acetyl-β-D-ribofuranosyl)imidazol in 50 ml über 4A Molekularsiebe getrocknetem DMF wird unter Rühren 23 Stunden unter Rückfluss bei 100°C mit 8,05 ml Methylisocyanat behandelt. Die Lösung wird zur Trockene eingedampft, zusammen mit 2 x 50 ml Methanol eingedampft und über Nacht unter Vakuum gelassen. 4,76 g des erhaltenen Schaums werden in 48 ml Aethylacetat/Chloroform (3/1) gelöst und über Silicagel mit Acetat/Chloroform (3/1) als Eluierungsmittel chromatographiert. Es werden 20 ml Fraktionen gesammelt. Die Fraktionen 122-160 werden kombiniert und zu einem weissen Schaum eingedampft. Zwecks Reinigung wird dieser Schaum in 10 ml Chloroform/Methanol (50/1) gelöst und über Silicagel mit Chloroform/Methanol (50/1) als Eluierungsmittel chromatographiert. Die 20 ml Fraktionen 100-140 werden zur Trockene eingedampft und man erhält 4-Cyan-5-[bis-(methylcarbamoyl)]-amino-1-(2', 3', 5'-tri-O-acetyl-β-D-ribofuranosyl)imidazol als weissen Schaum; IR 2245 $cm^{-1}$ (C≡N), 1753 (Ester), 1728, 1680 (Harnstoffcarbonyl); UV 227 nm (ε 11,360) in Methanol, 226 nm (ε 9540) in 0,1N HCl.

Eine Lösung von 480 ml 4-Cyan-5-[bis-(methylcarbamoyl)]-amino-1-(2', 3', 5-tri-O-acetyl-β-D-ribofuranosyl)-imidazol in 5 ml Methanol und 5 ml konzentriertem Ammoniumhydroxid wird 18 Stunden bei 5°C aufbewahrt. Das Produkt wird zur Trockene eingedampft und aus Aethanol/Wasser umkristallisiert. Man erhält 1-Methyl-isoguanosin vom Schmelzpunkt 267-268°C (Zersetzung).

## Beispiel 4

Eine Lösung von 200 mg Isoguanosin in 6 ml über 4A Molekularsiebe getrocknetem Dimethylsulfoxid wird 18 Stunden bei Raumtemperatur mit 215 mg Kaliumcarbonat und 0,088 ml Methyljodid gerührt. Das Gemisch wird filtriert, zu einem Oel eingedampft, letzteres in Methanol/Wasser (9/1) gelöst und auf 7 g Siliciumoxid gegeben. Das Siliciumoxid wird durch Verdampfung von 3 x 100 ml Aethanol getrocknet, über Nacht im Vakuum gelassen und auf eine Siliciumoxidsäule gegeben. Die Säule wird mit 750 ml Chloroform/Methanol (5/1) und dann mit Chloroform/Methanol (3/1) eluiert. Es werden die 20 ml Fraktionen 45-60 gesammelt und eingedampft. Man erhält rohes 2-Methoxy-adenosin. Nach Umkristallisation aus Wasser erhält man reines Produkt vom Schmelzpunkt 189-192°.

Die Fraktionen 100-140 werden zur Trockene eingedampft. Man erhält rohes 1-Methyl-isoguanosin. Ein Teil davon wird durch präparative Dünnschichtchromatographie auf Siliciumoxid mit Chloroform/Methanol (2/1) gereinigt. Die geeignete Portion wird mit Wasser extrahiert, der Extrakt zur Trockene eingedampft und in heissem Wasser gelöst. Kleine Mengen von Siliciumoxid werden durch Filtrierung entfernt. Das Filtrat wird zu 0,1 ml eingedampft und mit 0,2 ml Aethanol versetzt. Das Gemisch wird über Nacht bei 5°C gehalten und man erhält Kristalle vom Schmelzpunkt 265° C (Zersetzung).

## Beispiel 5

Tabletten der folgenden Zusammensetzung wurden in üblicher Weise hergestellt:

| | | |
|---|---|---|
| 1-Methyl-9β-D-ribofuranosyl-isoguanin | 50 | mg |
| Lactose | 95 | mg |
| Maisstärke | 100 | mg |
| Talkum | 4,5 | mg |
| Magnesiumstearat | 0,5 | mg |
| Totalgewicht | 250 | mg |

RAN 4008/295-K 0000770

## Patentansprüche

1. Verfahren zur Herstellung von 1-Methyl-isoguanosin, dadurch gekennzeichnet, dass man

a) es aus natürlichen Vorkommen in an sich bekannter Weise isoliert oder

b) eine Verbindung der Formel

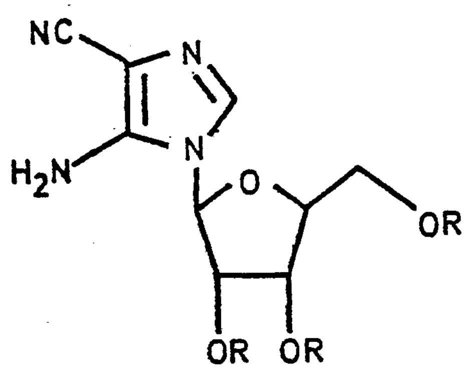

II

worin R eine leicht abspaltbare Acylgruppe darstellt, mit Methylisocyanat in Gegenwart eines stark aprotischen Lösungsmittels umsetzt und die erhaltene Verbindung der Formel

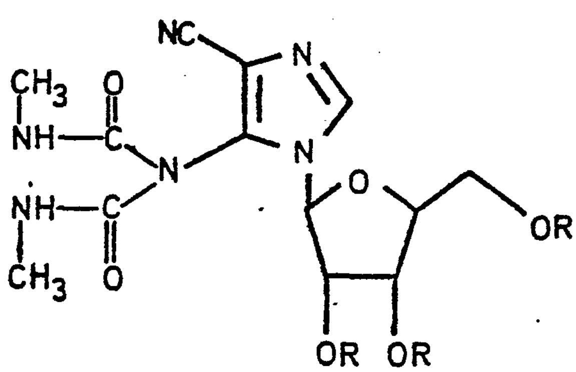

III

worin R obige Bedeutung hat, mit einer Base umsetzt oder

c) eine Verbindung der Formel

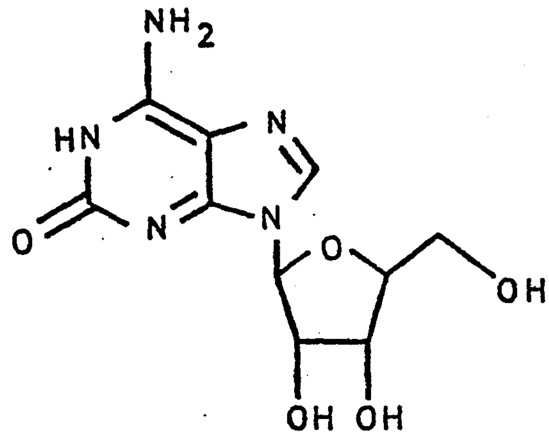

IV

mit einem Methylierungsmittel umsetzt.

2. Verfahren nach Anspruch 1, dadurch gekennzeichnet, dass man Verfahrensvariante a) durchführt.

3. Verfahren nach Anspruch 1, dadurch gekennzeichnet, dass man die Verfahrensvariante b) durchführt.

4. Verfahren nach Anspruch 1, dadurch gekennzeichnet, dass man die Verfahrensvariante c) durchführt.

5. Verfahren nach Anspruch 1 oder 2, dadurch gekennzeichnet, dass das natürliche Vorkommen ein Meeresorganismus ist und 1-Methyl-isoguanosin durch Extraktion mit einem Lösungsmittel und anschliessende Chromatographie isoliert wird.

6. Verfahren nach Anspruch 5, dadurch gekennzeichnet, dass das Lösungsmittel ein Gemisch von Wasser und Aethanol ist und der Extrakt durch Ionenaustauscherchromatographie gereinigt wird.

7. Verfahren nach Anspruch 1 oder 3, dadurch gekennzeichnet, dass die Acylgruppe R Acetyl ist.

8. Verfahren nach Anspruch 1 oder 4, dadurch gekennzeichnet, dass die Methylierung der Verbindung der Formel IV mit Methylhalogenid in Gegenwart eines aprotischen Lösungsmittels unter basischen Bedingungen durchgeführt wird.

9. Verfahren zur Herstellung von pharmazeutischen Präparaten, dadurch gekennzeichnet, dass man 1-Methyl-isoguanosin als wirksamen Bestandteil mit zur therapeutischen Verabreichung geeigneten nicht-toxischen inerten an sich in solchen Präparaten üblichen festen und flüssigen Trägern und/oder Excipientien vermischt.

10. Pharmazeutische Präparate, gekennzeichnet durch einen Gehalt an 1-Methyl-isoguanosin.

11. 1-Methyl-isoguanosin.

12. 1-Methyl-isoguanosin in praktisch reiner Form frei von natürlich vorkommenden Begleitstoffen.

13. Verwendung von 1-Methyl-isoguanosin als Muskelrelaxants und/oder als Mittel zur Beeinflussung des zentralen Nervensystems und/oder als antiinflammatorisches und/oder antiallergisches und/oder hypotensives Mittel.

14. Verbindungen der Formel

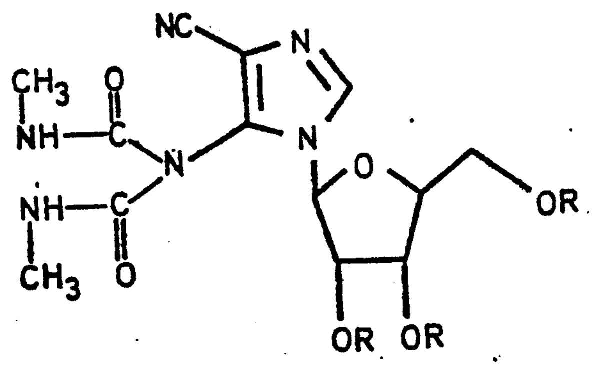

III

worin R eine leicht abspaltbare Acylgruppe darstellt.

15. Verbindung nach Anspruch 14 worin R Acetyl darstellt.